# EUROPEAN PATENT SPECIFICATION

(11) **EP 4 346 671 B1**
(45) Date of publication and mention of the grant of the patent: **18.11.2026**
(21) Application number: 22816757.3
(22) Date of filing: 01.06.2022
(51) Int. Cl.: A61B 18/14, A61B 17/32, A61B 18/00, A61B 5/01, A61B 5/00, A61N 1/40

(54) **METHOD AND APPARATUS FOR TREATING PRE-ULCERATIVE LESIONS WITH PULSED ELECTROMAGNETIC FIELDS**
VERFAHREN UND VORRICHTUNG ZUR BEHANDLUNG VON PRÄULZERATIVEN LÄSIONEN MIT GEPULSTEN ELEKTROMAGNETISCHEN FELDERN
PROCÉDÉ ET APPAREIL POUR TRAITER DES LÉSIONS PRÉ-ULCÉREUSES À L'AIDE DE CHAMPS ÉLECTROMAGNÉTIQUES PULSÉS

(30) Priority: 01.06.2021 US 202163195579 P
(43) Date of publication of application: 10.04.2024
(73) Proprietor: Regenesis Biomedical, Inc., Scottsdale, AZ 85250 (US)
(72) Inventor: EISIMINGER, Thomas I., Jr., Scottsdale, AZ 85250 (US); TASSONE, Erica, Scottsdale, AZ 85250 (US); ROBEY, Scott A., Scottsdale, AZ 85250 (US); AGUILAR, Donna, Scottsdale, AZ 85250 (US)
(74) Representative: Hancox, Jonathan Christopher
(86) International application number: PCT/US2022/031725
(87) International publication number: WO 2022/256375

(56) References cited:
- WO-A1-2010/124234
- WO-A1-2016/081952
- WO-A1-2018/089773
- US-A- 6 063 108

## Description

### BACKGROUND

Pulsed electromagnetic fields (PEMF) have been described for treating therapeutically resistant problems of both the musculoskeletal system as well as soft tissues. PEMF typically includes the use of low-energy, time-varying magnetic fields. For example, PEMF therapy has been used to treat non-union bone fractures and delayed union bone fractures. PEMF therapy has also been used for treatment of corresponding types of body soft tissue injuries including chronic refractory tendinitis, decubitus ulcers and ligament, tendon injuries, osteoporosis, and Charcot foot. During PEMF therapy, an electromagnetic transducer coil is generally placed in the vicinity of the injury (sometimes referred to as the "target area") such that pulsing the transducer coil will produce an applied or driving field that penetrates to the underlying tissue.

Treatment devices emitting magnetic and/or electromagnetic energy offer significant advantages over other types of electrical stimulators because magnetic and electromagnetic energy can be applied externally through clothing and wound dressings, thereby rendering such treatments completely non-invasive. Moreover, published reports of double-blind placebo-controlled clinical trials utilizing a RF transmission device (Diapulse) suggest that this ancillary treatment device significantly reduces wound healing time for open, chronic pressure ulcers as well as for surgical wounds. Studies using Dermagen, a magnetic device manufactured in Europe which produces a low frequency magnetic field, have demonstrated significant augmentation of healing of venous stasis ulcers.

Although PEMF has shown promise in treating existing ulcers, what is needed are methods and apparatuses to treat pre-ulcerous lesions. In particular it would be very beneficial to treat pre-ulcerous lesions early in their development, including before developing into visible sores.

WO 2018/089773 (Fractyl Laboratories, Inc.) discloses a method for performing a medical procedure in an intestine of a patient. The method comprises providing a system comprising: a catheter for insertion into the intestine, the catheter comprising: an elongate shaft comprising a distal portion; and a functional assembly positioned on the shaft distal portion and comprising at least one treatment element. The catheter is introduced into the patient, and target tissue is treated with the at least one treatment element. The target tissue comprises mucosal tissue of the small intestine, and the medical procedure can be configured to treat one or more diseases or disorders based on a measured physiologic parameter of the patient. WO 2016/081952 (Regenesis Biomedical, Inc.) relates to methods of modulating gene expression and biological regulatory pathways with pulsed electromagnetic field therapy for treating diseases or conditions associated with such gene expression or regulatory pathways that are susceptible to modulation by pulsed electromagnetic therapy, including diseases or conditions associated with inflammation, nerve pain and wound repair.

### Summary of the invention

The invention is defined in the appended claims. Aspects, embodiments and examples disclosed herein which do not fall within the scope of the appended claims do not form part of the invention, and are merely provided for illustrative purposes.

### SUMMARY OF THE DISCLOSURE

Described herein are pulsed electromagnetic field (PEMF) apparatuses (e.g., devices and systems, including PEMF therapy systems) and methods for treating pre-ulcerous lesions. The PEMF apparatuses described herein may include a PEMF delivery sub-system configured to generate a pulsed current signal, in conjunction with a detection (e.g., sensing, including but not limited to spectral or hyperspectral imaging, and/or thermal detection/imaging) sub-system that is coupled to the PEMF delivery sub-system; the PEMF sub-system may include one or more PEMF applicators coupled to the PEMF delivery sub-system for detecting a pre-ulcerous lesion and for delivering PEMF therapy specifically to a region including a pre-ulcerous lesion. In particular, the apparatuses described herein may determine if a patient has one or more pre-ulcerative lesions and may then provide a PEMF therapy to the pre-ulcerative lesions.

The PEMF therapy apparatus may determine the likelihood of a pre-ulcerous lesion (and/or the temperature of a pre-ulcerous lesion), in some cases by detecting the temperature of various locations of a patient. For example, the PEMF therapy apparatus may determine and track the temperatures of contralaterally-matched locations on the patient. If a temperature difference between the contralaterally-matched locations exceeds a threshold, then that location may include a pre-ulcerative lesion (e.g., the temperature differs by more than, 1 degree C, 1.5 degree C, 1.7 degrees C, 1.9 degrees C, 2 degrees C, 2.1 degrees C, 2.2 degrees C, 2.3 degrees C, 2.4 degrees C, etc.) compared to the contralateral region and/or an adjacent region, than the region may be considered as pre-ulcerous. In some cases, in addition or instead of comparing to a contralateral region, the body region may be compared to adjacent regions of the tissue and/or to a normalized standard. For example, if a region of tissue differs from an adjacent region by more than a threshold amount (e.g., more than, 1 degree C, 1.5 degree C, 1.7 degrees C, 1.9 degrees C, 2 degrees C, 2.1 degrees C, 2.2 degrees C, 2.3 degrees C, 2.4 degrees C, etc.) the region may be considered as including a pre-ulcerous lesion. In some examples, the temperature of one or more tissue regions may be compared to a standard value, including after normalizing, e.g., based on an estimated core body temperature and/or peripheral body temperature, as measured from an extremity such as the hand (fingers, back of hand, etc.), arm, ears (earlobe, etc.), leg, foot (toes, heel, etc.), or the like.

In some examples the apparatus or method my use tissue oximetry (also known as hyperspectral tissue oximetry) to detect or identify pre-ulcerous lesions and may apply PEMF to treat these regions to prevent and/or reverse wound development and progression. Tissue oximetry may detect changes in blood oxygenation as a proxy for determining pre-ulcerous lesions by determining the reflected and/or absorbed light at particular wavelengths in order to assess oxyhemoglobin, deoxyhemoglobin, and/or oxyhemoglobin saturation in the superficial tissue (e.g., skin).

The PEMF apparatus (and in particular, the PEMF delivery sub-system) may provide a pulsed electromagnetic field signal to the PEMF applicator. The PEMF applicator may emit pulsed electromagnetic fields (for example, magnetic fields) toward the patient regions with the pre-ulcerative lesions. The electromagnetic fields may inhibit and/or prevent the pre-ulcerative lesions from erupting and reduce inflammation. This delivery may be specifically triggered, calibrated and/or targeted based on the detection of a pre-ulcerous lesion using the detection sub-system.

One aspect of subject matter described herein may be implemented in a method for treating pre-ulcerative lesions. A method may include performing a first scan to determine optical properties and/or temperatures associated with one or more body regions of a patient, determining a location of a pre-ulcerative lesion based on the first scan, and delivering a first pulsed electromagnetic field (PEMF) treatment to the determined location of the pre-ulcerative lesion. The pre-ulcerative lesion may include an unerupted diabetic foot ulcer, an unerupted pressure ulcer, a venous leg ulcer, or a combination thereof. Furthermore, performing the first scan may include determining optical properties and/or temperatures of two or more body locations. In some examples, determining the location may include determining an optical property and/or temperature difference between two or more contralaterally-matched body locations. In some examples, the determined difference in optical property and/or temperature may be greater than a threshold. In some examples, determining the location may include determining the location based on adjacent tissue region(s). In some examples, determining the location may include normalizing the optical property and/or temperature scan based on a measurement from a separate body region (e.g., contralateral body region and/or peripheral body region and/or core body temperature, etc.).

In some examples, the PEMF treatment (e.g., first treatment) may include a first time duration, a first number of treatments per day, and a first pulsed energy signal strength. In some other examples, the method may include performing a second scan of the body locations and delivering a second PEMF treatment based at least in part on the second scan (e.g., an optical property and/or thermal scan). Furthermore, the second scan may show an increase in a difference (e.g., of optical property and/or thermal properties) between two or more contralaterally-matched body locations, with respect to the first scan. Still further, the second PEMF treatment may include a longer time duration, an increased number of treatments per day, or an increased pulsed energy signal strength, with respect to the first PEMF treatment. As used herein a scan may include a thermal image and/or one or more optical detections and/or images (e.g., taken at wavelengths that reflect the development of a pre-ulcerous lesion, as described herein). In some examples the scan does not require or include imaging.

In some examples, the second scan may show a decrease in a difference between two or more contralaterally-matched body locations, with respect to the first scan. Furthermore, the second PEMF treatment may include a shorter time duration, a decreased number of treatments per day, or a decreased pulsed energy signal strength, compared to the first PEMF treatment. In some other examples, the first scan may be performed via a foot monitoring mat (e.g., temperature and/or optical property monitoring mat), a conforming sensing mat, a camera, or a combination thereof.

In some examples described herein a pulsed electromagnetic field (PEMF) systems includes a thermal detection sub-system configured to perform a first thermal scan to determine temperatures associated with body locations of a patient, a PEMF delivery sub-system coupled to the thermal detection sub-system, and one or more applicators. Any of these apparatuses (e.g., PEMF systems) may include a controller with one or more processors configured to determine a location of a pre-ulcerative lesion based on the first thermal scan, and to determine and coordinate delivery of a dose for the PEMF treatment to the determined location of the pre-ulcerative lesion.

The pre-ulcerative lesion may include an unerupted diabetic foot ulcer, an unerupted pressure ulcer, a venous leg ulcer, or a combination thereof. Furthermore, the PEMF apparatus may be configured to determine temperatures and/or optical properties of two or more body locations. For example, a PEMF apparatus may be configured to determine a temperature difference between two or more contralaterally-matched body locations. The determined temperature difference may be greater than a threshold (e.g., greater than about 1 degree C, 1.1 degrees C, 1.2 degrees C, 1.3 degrees C, 1.4 degrees C, 1.5 degrees C, 1.6 degrees C, 1.7 degrees C, 1.8 degrees C, 1.9 degrees C, 2.0 degrees C, 2.1 degrees C, 2.2 degrees C, 2.3 degrees C, 2.4 degrees C, 2.5 degrees C, etc.).

The first PEMF treatment may include a first time duration, a first number of treatments per day, and a first pulsed energy signal strength. In some examples, a thermal detection sub-system may be configured to perform a second (or more) thermal scans of the body locations and the PEMF apparatus may be configured to deliver a second PEMF treatment based at least in part on the second thermal scan. The second thermal scan may show an increase in a temperature difference between two or more contralaterally-matched body locations, with respect to the first thermal scan. Further, the second PEMF treatment may include a longer time duration, an increased number of treatments per day, or an increased pulsed energy signal strength, with respect to the first PEMF treatment.

In some examples, the second thermal scan may show a decrease in a temperature difference between two or more contralaterally-matched body locations, with respect to the first thermal scan. Further, the second PEMF treatment may include a shorter time duration, a decreased number of treatments per day, or a decreased pulsed energy signal strength, with respect to the first PEMF treatment. The thermal detection sub-system may be configured to perform thermal scans via a foot temperature monitoring mat, a conforming temperature sensing mat, a thermal camera, or a combination thereof.

Any of the apparatuses and methods described herein may be implemented as a non-transitory computer-readable storage medium storing instructions that, when executed by one or more processors of a pulsed electromagnetic field (PEMF) apparatus, cause the apparatus to perform a first scan to determine temperatures and/or optical properties associated with body locations of a patient, determine a location of a pre-ulcerative lesion based on the first scan, and deliver a first PEMF treatment to the determined location of the pre-ulcerative lesion.

In some examples, the pre-ulcerative lesion includes an unerupted diabetic foot ulcer, an unerupted pressure ulcer, a venous leg ulcer, or a combination thereof. In some other examples, execution of the instructions to perform the first thermal scan may cause the system to further determine temperatures of two or more body location. In still other examples, execution of the instructions to determine the location may cause the system to further determine a temperature and/or optical property difference between two or more contralaterally-matched body locations. The determined temperature difference may be greater than a threshold. In some examples, the first PEMF treatment may include a first time duration, a first number of treatments per day, and a first pulsed energy signal strength.

In some examples, execution of the instructions may cause the system to perform a second scan of the body locations and deliver a second PEMF treatment based at least in part on the second scan. The second scan may be a thermal and/or optical property scan. For example, the second scan may show an increase in a temperature difference between two or more contralaterally-matched body locations, with respect to the first thermal scan. The second PEMF treatment may include a longer time duration, an increased number of treatments per day, or an increased pulsed energy signal strength, with respect to the first PEMF treatment.

In some examples, the second scan may be a thermal scan that shows a decrease in a temperature difference between two or more contralaterally-matched body locations, with respect to the first thermal scan. Further, the second PEMF treatment may include a shorter time duration, a decreased number of treatments per day, or a decreased pulsed energy signal strength, with respect to the first PEMF treatment. Still further, the second PEMF treatment may include a shorter time duration, a decreased number of treatments per day, or a decreased pulsed energy signal strength, with respect to the first PEMF treatment.

For example, described herein are methods of treating pre-ulcerative lesions, the method comprising: performing a scan to determining a location of a pre-ulcerative lesion prior to ulcer formation; and delivering a first pulsed electromagnetic field (PEMF) treatment to the determined location of the pre-ulcerative lesion. The scan may comprise a thermal scan, a tissue oximetry scan, or a combination of the two. The pre-ulcerative lesion may be an unerupted diabetic foot ulcer, an unerupted pressure ulcer, a venous leg ulcer, or a combination thereof.

Determining the location may include determining a difference between scans of two or more contralaterally-matched body locations. For example, determining the difference may comprise determining a temperature difference is greater than a threshold. The PEMF treatment may comprise applying 27.12 MHz pulses having a pulse duration of between about 35-50 microseconds (e.g., about 42 microseconds) and delivered at between about 800-1200 (e.g., about 1000) times per second.

Any of these methods may include performing a second (or more) scan(s) of the body including (or limited to) the identified location and delivering additional PEMF treatment based at least in part on the additional scans. The additional scan(s) may comprise a thermal scan showing an increase or a decrease in a temperature difference between two or more contralaterally-matched body locations, with respect to the first thermal scan. If the scan(s) indicate an increase in the temperature difference, the additional PEMF treatment(s) may include one or more of: an increased treatment duration, an increased number of treatments per day, or an increased pulsed energy signal strength, with respect to the first PEMF treatment; if the scan(s) indicate a decrease in the temperature differences, the additional PEMF treatment(s) may include one or more of: a decreased treatment duration, a decreased number of treatments per day, or a decreased pulsed energy signal strength, with respect to the first PEMF treatment.

The scans may be performed via a camera (e.g., to detect the tissue oximetry), a foot temperature monitoring mat, a conforming temperature sensing mat, a thermal camera, or a combination thereof. Any of the methods or apparatuses for detecting tissue oximetry may include emitters within the target wavelength range (e.g., between 450 and 700 nm, between 500-650 nm, etc.) and filters and/or receiver(s) to detect reflected and/or emitted light.

For example, a method for treating pre-ulcerative lesions may include: performing a scan to determining a location of a pre-ulcerative lesion prior to ulcer formation on a patient's foot; and delivering a pulsed electromagnetic field (PEMF) treatment to the determined location of the pre-ulcerative lesion, wherein the treatment comprises applying 27.12 MHz pulses lasting between 35-50 microseconds and delivered at between 800-1200 times per second at least once per day.

Also described herein are pulsed electromagnetic field (PEMF) system comprising: a detection sub-system configured to perform a first scan to determine a location of a pre-ulcerative lesion prior to ulcer formation; a PEMF generator configured to generate a PEMF treatment output; and a PEMF applicator coupled to the PEMF generator and configured to deliver a PEMF treatment to the determined location of the pre-ulcerative lesion.

Any of these PEMF systems may include a processor configured to monitor the pre-ulcerative lesion over time and to adjust the PEMF treatment based on a progression of the pre-ulcerative lesion. The detection sub-system may comprise a tissue oximetry detection sub-system and/or a thermal detection sub-system. The thermal detection sub-system may be configured to determine temperatures of two or more body locations. The thermal detection sub-system may be configured to determine a temperature difference between two or more contralaterally-matched body locations. The determined temperature difference may be greater than a threshold (e.g., 5% or more, 10% or more, 15% or more, 20% or more, 25% or more, 30% or more, 35% or more, 40% or more, 45% or more, 50% or more, 55% or more, 60% or more, 70% or more 75% or more, 80% or more, 85% or more, etc.).

The PEMF generator may be configured to generate 27.12 MHz pulses lasting between 35-50 microseconds (e.g., having a pulse duration of about 42 microseconds) and delivered at between 800-1200 (e.g., about 1000) times per second. The detection sub-system may comprise one or more of: a foot temperature monitoring mat, a conforming temperature sensing mat, a thermal camera.

For example, a pulsed electromagnetic field (PEMF) system may include: a detection sub-system configured to perform a first scan to determine a location of a pre-ulcerative lesion prior to ulcer formation; a PEMF generator configured to generate a PEMF treatment comprising 27.12 MHz pulses having a pulse duration of between 35-50 microseconds and delivered at between 800-1200 times per second; a PEMF applicator coupled to the PEMF generator and configured to deliver the PEMF treatment to the determined location of the pre-ulcerative lesion; and a processor configured to receive input from the detection sub-system and to control the application of the PEMF treatment from the PEMF applicator.

All of the methods and apparatuses described herein, in any combination, are herein contemplated and can be used to achieve the benefits as described herein.

### BRIEF DESCRIPTION OF THE DRAWINGS

A better understanding of the features and advantages of the methods and apparatuses described herein will be obtained by reference to the following detailed description that sets forth illustrative embodiments, and the accompanying drawings of which:
FIG. 1A schematically illustrates a PEMF apparatus as described herein.
FIG. 1B schematically illustrates a PEMF apparatus as described herein.
FIG. 1C schematically illustrates a PEMF apparatus as described herein.
FIG. 2 is a flowchart depicting an example of one method for detecting and treating a patient having at least one body region in a pre-ulcerative condition.
FIG. 3 shows a block diagram of one example of a PEMF therapy apparatus.
FIG. 4 is a table illustrating results of a generally applicable gene-set enrichment (GAGE) analysis of RNA sequence data.
FIG. 5 is a graph showing the results of a principle component analysis of RNA sequence data.

### DETAILED DESCRIPTION

Ulcerative lesions are wounds that may erupt on a patient's body. Some patients, such as patients diagnosed with diabetes, may be more prone to ulcers and ulcerative lesions, including, but not limited to, diabetic foot ulcers (DFU), pressure ulcers, venous leg ulcers, and other traumatic wounds. For some patients, the ulcerative lesions may be prevented through the application of pulsed electromagnetic fields (PEMF). PEMF therapy may be administered through a PEMF therapy device coupled to one or more PEMF applicators. The PEMF applicators may be configured to radiate electromagnetic fields, including magnetic fields, into selected body regions where one or more ulcerative lesions are expected or predicted to erupt. The administration of PEMF therapy into the selected body regions may calm the surrounding tissues and inhibit the eruption of the lesions. The ulcerative lesions that are expected or predicted to erupt may be referred to as pre-ulcerative lesions.

In some examples pre-ulcerative lesions may be predicted using an optical and/or thermal detection sub-system. For example, a comparison of the temperatures of contralaterally-matched body locations (e.g., matched locations on opposite sides of the body) may be used to determine likely body regions where an ulcerative lesion may occur. PEMF therapy may be administered to these body regions to prevent ulcerative lesions from erupting or otherwise occurring. Furthermore, in some examples, the PEMF therapy that is delivered may be determined, at least in part, by the temperatures associated with these body locations. Alternatively or additionally, optical sub-systems may include tissue oximetry using one or more wavelengths of light.

In general, described herein are methods and apparatuses for the treatment of a pre-ulcerative lesion by targeted application of pulsed electromagnetic fields (PEMF) to an identified area determined to be likely to develop an ulcer (e.g., a diabetic foot ulcer). For example, diabetic foot ulcers (DFU) are a common sequela of diabetes, occurring in 2-6% of patients annually. One of the primary modalities to predict ulceration is inflammation, however, clinical signs of inflammation are difficult to detect visually both by patients and health care providers. The methods and apparatuses described herein may detect signs of inflammation using, e.g., skin temperature measurements or optical detection (e.g., hyperspectral imaging), which may alert patients and/or physicians of a pre-ulcerative lesion. Once the pre-ulcerative lesion is identified, it may be treated to reduce inflammation through indirect methods such as offloading or prescription shoes; however it would be particularly beneficial to provide a direct therapy to treat the identified areas. Surprisingly, it the inventors have found that treatment with PEMF may prevent or eliminate the progression of pre-ulcerous lesions into ulcers, as will be described in greater detail herein. This is surprising because prior work has found that although PEMF may be helpful improving recovery time and accelerating healing of skin ulcer lesions, PEMF appears to be helpful only with open lesions, e.g., prior to wound closure. In contrast, it has been suggested that PEMF instead inhibits the remodeling phase, e.g., after wound closure, and may inhibit collagen remodeling.

In contrast the results descried herein instead show that prior to disruption of the wound (ulcer), during a period more akin to the remodeling phase after wound closure, the application of PEMF early may instead reduce or eliminate the pre-ulceration, preventing the wound from forming. In addition to preliminary experiments showing the efficacy of treated regions identified as per-ulcerous using a detection method as described herein, a retrospective analysis of patient data shows that, among patients treating with PEMF therapy for diabetic neuropathy, a significantly lower rate of diabetic foot ulcers (DFU) occurred, particularly given the prevalence of DFU among diabetic patients. Thus, described herein are methods and apparatuses for monitoring areas of concern (e.g., feet) for pre-ulcerative lesions and treating the identified area with PEMF directed at the lesion.

For example, diabetic patients may be prone to having ulcerous lesions, particularly in the region of the patient's feet. If a lesion is expected or predicted to occur within the patient's feet (for example, as detected with a temperature monitoring foot mat), PEMF therapy may be provided to the patient's feet to promote healing and prevent the lesion from erupting.

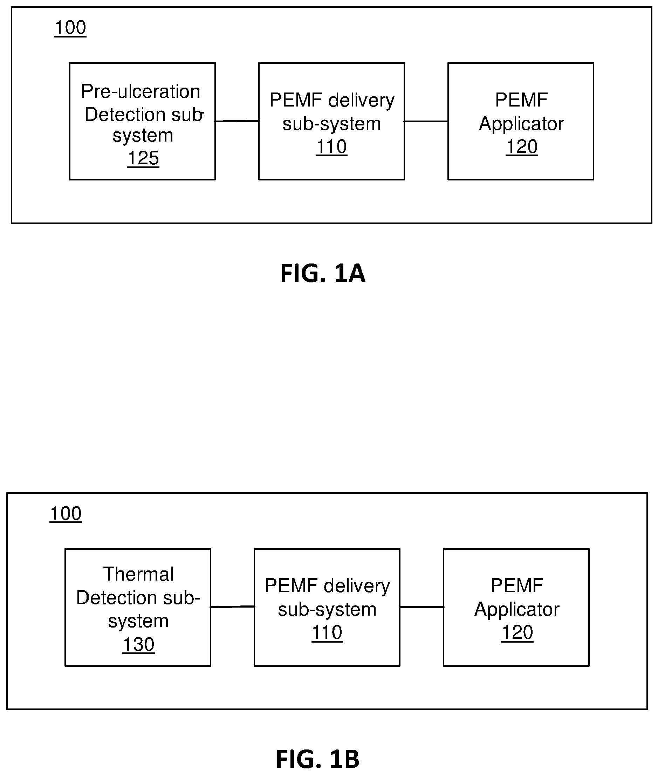

FIG. 1A is a diagram of an example of a PEMF apparatus 100, according to some examples. The PEMF system 100 may include a PEMF delivery sub-system 110, a PEMF applicator 120, and a pre-ulceration detection sub-system 125. The pre-ulceration detection system or sub-system may generally be referred to herein as a pre-ulceration detector. The PEMF delivery sub-system 110 may be used to deliver one or more high-power, pulsed electromagnetic fields to a patient through one or more PEMF applicators, such as the PEMF applicator 120. Although only one PEMF applicator 120 is shown, in other examples, the PEMF system 100 may include multiple PEMF applicators 120. The pulsed electromagnetic fields may provide a therapeutic effect to the patient in a non-invasive manner. In some examples, the pulsed electromagnetic fields may upregulate cytokines, collagen, alpha SMA, FGF and other markers associated with wound healing. In still other examples, the pulsed electromagnetic fields may treat inflammation and tissue remodeling associated with a predicted or pending diabetic foot ulcers and/or pressure ulcers.

### Optical Detection

In any of the methods and apparatuses described herein the pre-ulceration detector (e.g., pre-ulceration detection sub-system) may be an optical detection system or sub-system that identifies region of pre-ulcerous lesions, e.g., regions where it is likely that an ulcerous lesion will erupt, based on an optical property of the tissue. For example, the pre-ulceration detector may be configured to detect pre-ulcerations in one or more regions of the tissue, including in particularly the feet and/or legs, using an optical sensor such as a tissue oximetry sensing sub-system or system. In some example, the pre-ulceration detector may be an optical detector configured to sense absorption and/or reflectance of light in one or more specific wavelengths (e.g., between abut 500 and 650 nm) to assess the risk of ulcer development, including (but not limited to) diabetic foot ulcer development. For example, tissue oximetry may identify ischemic and inflammatory region before they are visible during a clinical examination. Any of these pre-ulceration detectors may record a series of images representing the intensity of diffusely reflected light from the tissue (e.g., feet) at discrete wavelengths. The resulting set of images may include the reflectance spectrum of the tissue across a tissue surface, e.g., corresponding to the surface of the tissue (e.g., feet). These images may be examined by the system/sub-system (generically, "detector") to identify chromophores, such as melanin or hemoglobin (oxyhemoglobin and deoxyhemoglobin). The ratio of oxyhemoglobin concentration to the total hemoglobin concentration in the blood is referred to as oxygen saturation (SO₂). It indicates the rate of oxygen delivery to and consumption by the tissues. The optical extinction coefficient of oxyhemoglobin may be distinguished from that of deoxyhemoglobin, and a spectral absorption coefficient of tissue for different tissue regions may be based on the concentration and oxygen saturation of hemoglobin within these regions of tissue. Changes in the tissue's spectral absorption coefficient may change the diffuse reflectance spectrum for this region of tissue. Thus, transcutaneous tissue oximetry may permit estimate of the hemoglobin concentration and oxygen saturation based on the diffuse reflectance of the tissue.

For example, imaging in the visible and near-infrared parts of the spectrum may be used to determine the spatial distribution of oxygen saturation in skin, to detect the circulatory changes in the diabetic foot. Oxyhemoglobin and deoxyhemoglobin concentrations as well as oxygen saturation may be calculated from the tissue reflectance and/or absorbance. Changes in either or both oxyhemoglobin and deoxyhemoglobin concentrations relative to adjacent and/or contralateral regions may be used to determine the likelihood of developing an ulcer, including a diabetic foot ulcer.

### Temperature Detection

Alternatively or additionally, a pre-ulceration detector may include a temperature-based ulceration detector. FIG. 1B is a diagram of an example of a PEMF apparatus 100, according to some examples. The PEMF system 100 may include a PEMF delivery sub-system 110, a PEMF applicator 120, and a thermal detection sub-system 130. The PEMF delivery sub-system 110 may be used to deliver pulsed electromagnetic fields to a patient through one or more PEMF applicators, such as the PEMF applicator 120. Although only one PEMF applicator 120 is shown, in other examples, the PEMF system 100 may include more than one PEMF applicators 120. The pulsed electromagnetic fields may provide a therapeutic effect to the patient in a non-invasive manner. In some examples, the pulsed electromagnetic fields may upregulate cytokines, collagen, alpha SMA, FGF and other markers associated with wound healing. In still other examples, the pulsed electromagnetic fields may treat inflammation and tissue remodeling associated with a predicted or pending diabetic foot ulcers and/or pressure ulcers.

The temperatures may be determined with any feasible thermal detection sub-system. In one example, a temperature monitoring foot mat may be used to determine the temperature of contralaterally-matched plantar locations to predict likely locations of diabetic foot ulcers. A temperature monitoring mat may include a surface having an array of temperature-sensing elements (e.g., thermistors, etc.) configured to detect temperature of the tissue placed thereon. In other examples, an optical thermal detection sub-system or other temperature sensing device may be used to detect the temperatures of any feasible body parts.

The thermal detection sub-system 130 may determine temperatures associated with various body regions, particularly body regions that may be candidates for predicted ulcerous lesions. In some examples, the thermal detection sub-system 130 may perform thermal scans that can monitor contralaterally-matched body locations. For some patients, a pre-ulcerative condition may be indicated by a temperature differential between two contralaterally-matched body locations that is greater than a threshold.

In one example, the thermal detection sub-system 130 may be a foot temperature monitoring mat. The patient may stand on the mat and the PEMF therapy device 110 may determine the temperature of various positions of the patient's feet. In particular, the PEMF therapy device 110 may compare two or more contralateral locations of the patient's feet. If the difference in temperature between the two contralateral locations is greater than a threshold (for example, greater than 2.22 degrees Celsius or 4.0 degrees Fahrenheit), then the PEMF therapy device 110 may determine that that location of the foot may be in a pre-ulcerative condition.

In another example, the thermal detection sub-system 130 may be a conforming thermal sensing mat. The conforming thermal sensing mat may be wrapped around any feasible portion of the patient's body in order to sense (e.g., determine) the temperature of various body locations. Using the conforming thermal sensing mat, the PEMF therapy apparatus 100 may determine two or more contralateral locations that may have a temperature difference greater than a threshold. Those locations may be a location of the body that may be in a pre-ulcerative condition. A similar analysis may be used for the optical (e.g., spectral) analysis described above.

In yet another example, a thermal detection sub-system 130 may be a thermal imaging camera. The thermal imaging camera may be used to determine a temperature profile of any feasible portion of the patient. Thus, the PEMF therapy apparatus 100 may use the thermal imaging camera to determine two or more contralateral locations of the body may have a temperature difference greater than a threshold. Those locations may be a location of the body that may be in a pre-ulcerative condition.

After determining the location of a pre-ulcerative condition (e.g., a pre-ulcerative lesion, or the like), the PEMF therapy apparatus 100 may, using the PEMF delivery sub-system, deliver a PEMF treatment through the PEMF applicator 120. For example, the PEMF applicator 120 may be placed adjacent to (and in some cases, in contact with) the location of the body that may be in a pre-ulcerative condition. The PEMF delivery sub-system 110 may then provide an appropriate pulsed energy signal to the PEMF applicator 120. The PEMF apparatus 100 may include a controller, including one or more processors for determining the presence of a pre-ulcerous lesion (based on input from a thermal detection sub-system and/or an optical detection sub-system or a hybrid thermal/optical property detection sub-system) and may generate an appropriate dose by controlling the PEMF delivery sub-system to be delivered by the one or more applicators. In some examples the controller is included as part of the PEMF delivery sub-system; in other examples the controller may be separate or partially separate from the PEMF delivery sub-system.

Although many of the examples descried herein include a pre-ulceration detection subsystem 125, in some examples the detection sub-system may be separate and/or a generic detection sub-system may be used. In examples using a thermal pre-ulceration detection sub-system, the thermal data (including, but not limited to thermal imaging data) may be provided to the apparatus and the data may be used as described herein. Alternatively in some examples using an optical property detection sub-system (e.g., a spectral imaging sub-system), the data may be provided to the apparatus and used as described herein.

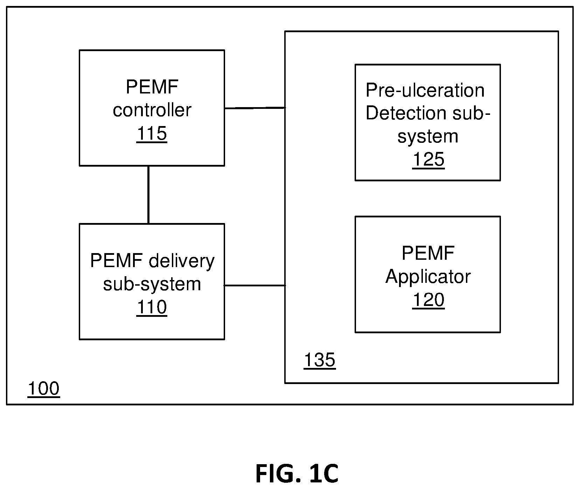

Further, in some examples, as shown schematically in FIG. 1C, the pre-ulceration detection sub-system may be combined with (e.g., integrated together with) the applicator. For example, in FIG. 1C, the apparatus 100 includes a controller 115, a PEMF delivery sub-system 110 (which may include, e.g., the waveform generator, timer circuitry, power handling/conditioning components, etc.), and a patent interface 135 that includes both the applicator 120 and detection sub-system 125. The patient interface may be configured as a pad or wrap having a patient-contacting surface for direction or indirectly contacting the patient's body, e.g., though a sock or bandage, etc. The patient interface may include the thermal detection sub-system, which may include an array or thermal sensors, integrated into the PEMF applicator(s). For example, multiple PEMF applicators may be arranged on the patient interface and may overlap with the thermal detection sub-system, which may detect one or more pre-ulcerative lesions within the tissue; the one or more PEMF applicators corresponding to the locations of the one or more pre-ulcerative lesions may be activated during treatment and may be controlled by the controller 115.

In general, the PEMF applicators 120 may be any feasible electromagnetic transducer. In some examples, the pulsed energy signal may be a high-power pulsed electromagnetic field signal may have a carrier frequency in the MHz range. For example, the carrier frequency may be between about 6 MHz and 100 MHz (e.g., about 27 MHz, about 10 MHz, between about 10 MHz and 60 MHz, etc.). The pulsed energy signal may cause the PEMF applicator 120 to emit a magnetic field that may penetrate the body. The magnetic field may treat tissues, including tissues in a pre-ulcerative condition, that may be underneath a closed epidermis. The pulsed energy signal may prevent an ulcer from erupting from the tissues in the pre-ulcerative condition. In some examples, the pulsed energy signal may additionally, or in the alternative, decrease an inflammatory response. In another example, the pulsed energy signal may reduce symptoms associated with peripheral neuropathy.

In some examples, the one or more PEMF applicators 120 (and/or the patient interface 135 including the one or more applicators) may be shaped to conform and/or treat particular body areas. For example, the PEMF applicator 120 may be shaped to receive and/or contact a patient's feet, similar to the foot temperature monitoring mat described above. In other examples, the PEMF applicator 120 may be shaped to conform to a hand, an arm, or any other feasible body part.

In some examples, the PEMF apparatus 100 may perform a first or baseline scan (e.g., a thermal, e.g., temperature, scan and/or an optical property scan). The baseline scan may indicate or otherwise determine a body location that is in a pre-ulcerative condition through a difference between two contralaterally-matched body locations, adjacent region(s) and/or average regions. A predetermined time period after a PEMF treatment is delivered to a patient, a second (e.g., subsequent) scan may be performed. The PEMF apparatus 100 (e.g., via a controller including one or more processors) may compare results of the second scan to the baseline scan. If a difference between the two locations, e.g., contralateral locations, in the subsequent scan (e.g., the contralateral locations that were also included in the baseline scan) is no longer present, then the PEMF treatments may be suspended, as the pre-ulcerative condition may no longer exist. On the other hand, if the difference between the two contralateral locations in the subsequent thermal scan remains, then a subsequent PEMF therapy treatment may be scheduled and/or performed. In some cases, the subsequent PEMF therapy treatment may be increased in terms of duration and/or electromagnetic field strength. For example, if a subsequent thermal scan shows an increase in temperature differential between the two contralateral body locations, or the subsequent thermal scan shows little or no reduction in temperature differential between two contralateral body locations compared to the baseline thermal scan, then the subsequent PEMF therapy treatment may be increased in duration and/or electromagnetic field strength. Similar techniques may be used with optical properties (e.g., related to oxygenation of the tissue).

In general, the apparatuses described herein may include the controller and one or more processors which may be configured to identify one or more pre-ulcerative lesions, and/or determine a dose and/or delivery the dose (including targeted delivery to the pre-ulcerative lesion). In some examples, all or some of the processing for identifying, determining dose and/or coordinating the PEMF dose, may be at least partially handled remotely. Thus any of these apparatuses may include a wired or wireless circuitry that may communicate with a remote processor. Even in variations in which all or some of the steps of identifying one or more pre-ulcerative lesions, determining a dose and/or delivering the dose are done locally, the apparatus may communicate, confirm, and/or report this data to a remote server. In particular, a remote server may be used for patient verification and the like. For example, a remote server may store historical treatment data (the first thermal scan data and/or treatment dose(s)) for comparison with the later thermal scan data and/or treatments.

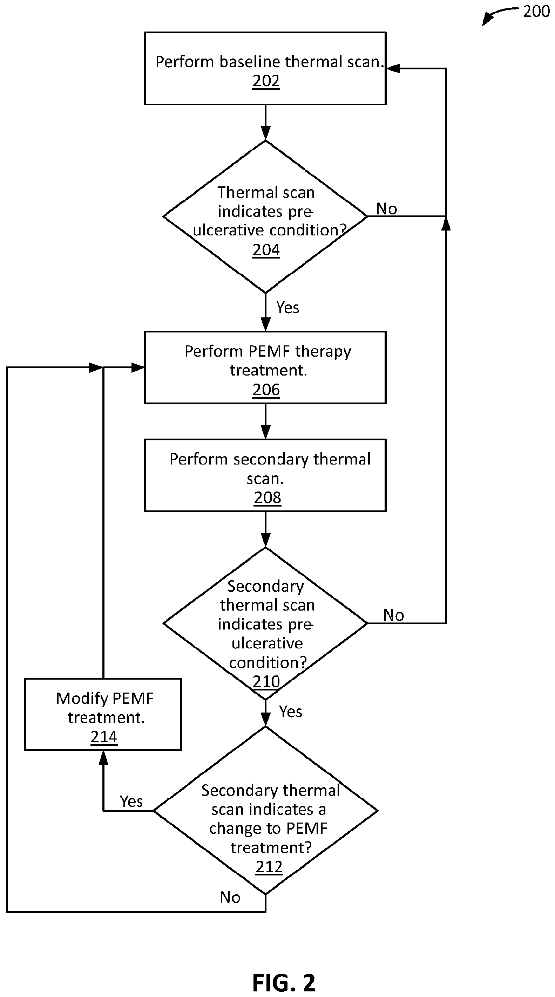

FIG. 2 is a flowchart depicting an example of one method 200 for detecting and treating a patient having at least one body region in a pre-ulcerative condition. Some examples may perform the operations described herein with additional operations, fewer operations, operations in a different order, operations in parallel, and some operations differently. The operations herein are described as being performed by the PEMF apparatus 100 of FIG. 1B or 1C for ease of explanation. These operations can be performed by any feasible device or processor that may be configured to receive and/or detect the conditions described herein and perform and/or deliver the therapies described herein. For example, these apparatuses may be used with optical property detection and/or thermal detection (or in some cases a combination of both) to detect per-ulcerative regions. In FIG. 2 the example includes thermal detection, but it should be explicitly understood that other detection technique (including optical property techniques) may be used.

In FIG. 2, the method 200 may begin as the PEMF therapy device 110 performs a baseline thermal scan 202. For example, the PEMF therapy device 110 may scan the temperature of one or more body regions with the thermal detection sub-system 130. The thermal detection sub-system 130 may be a temperature monitoring foot mat, a conforming thermal sensing mat, a thermal imaging camera, or any other technically feasible temperature sensing device. The baseline thermal scan may include two or more contralateral locations of the patient's body and/or adjacent tissue regions, and/or other body regions (including other peripheral body regions).

Next, the PEMF apparatus 100 determines if the thermal scan indicates a pre-ulcerative condition 204. For example, the PEMF apparatus 100 may determine if a temperature difference between two or more contralateral body locations is greater than a threshold temperature and, therefore, may indicate a pre-ulcerative condition. In some examples, the threshold temperature may be, e.g., greater than about 1.5 degrees Celsius (e.g., greater than about 1.6 degrees C, 1.7 degrees C, 1.8 degrees C, 1.9 degrees C, 2.0 degrees C, 2.1 degrees C, 2.2 degrees C, 2.3 degrees C, 2.4 degrees C, 2.5 degrees C, etc. for the feet) such as greater than about 2.22 degrees Celsius or 4.0 degrees Fahrenheit. In general, the threshold temperature may be any feasible temperature difference, and may be related to the body region. Pre-ulcerative conditions may include unerupted diabetic foot ulcers, unerupted pressure ulcers, venous leg ulcers, or any other feasible condition.

If the PEMF apparatus 100 confirms that no pre-ulcerative condition exists, then the method returns to 202. On the other hand, if the PEMF apparatus 100 determines that a pre-ulcerative condition exists, then a PEMF therapy treatment is performed 206. For example, the PEMF delivery sub-system 110 may provide a pulsed energy signal to the PEMF applicator 120. The PEMF applicator 120 may, in turn, emit a therapeutic electromagnetic field toward a body region determined to be in a pre-ulcerative condition. In some cases, the PEMF therapy treatment may be a twice daily treatment including a standard or default dose (e.g., standard pulsed energy signal strength) where each treatment has a duration of thirty (30) minutes. As mentioned, the apparatus may customize the dosage and/or the location based on the detected pre-ulcerous lesion. The PEMF therapy treatment may thereby prevent an eruption of ulcers in the determined body region. Thus, the PEMF therapy treatment may prevent diabetic foot ulcers, pressure ulcers, venous leg ulcers, or the like from erupting through the skin.

Next, the PEMF apparatus 100 may perform a secondary thermal scan 208. The secondary thermal scan may be performed via the thermal detection sub-system 130. The secondary thermal scan 208 may be used to determine if the pre-ulcerative condition has remained the same, is reducing, or is increasing. Typically, the secondary thermal scan 208 may be performed after a predetermined time period following the PEMF therapy treatment of 206. The secondary thermal scan 208 may include the same body locations, including the same contralateral body locations as the baseline thermal scan. The time period may be hours (e.g., between 8-12 hours, between 12-24 hours, etc.), days (between 1-7 days, between 3-14 days,, between 1-14 days, between 7-21 days, etc.), or months (e.g., between 1-2 months, between 1-3 months, etc.).

The PEMF apparatus 100 may determine if the secondary thermal scan indicates a pre-ulcerative condition 210. For example, the PEMF apparatus 100 may determine if the contralateral body locations continue to have a temperature difference greater than a threshold. If the temperature difference is not greater than a threshold, then PEMF treatment may end, and the method returns to 202.

On the other hand, if the PEMF apparatus 100 determines that a pre-ulcerative condition exists, then the PEMF apparatus 100 may determine if the thermal scan indicates a change to the PEMF treatment 212. Indicators that the PEMF treatment may need updating or modification may include a change in the determined temperature difference between contralateral body locations. For example, if the temperature difference increases, then the PEMF treatment may be increased in terms of frequency (e.g., a number of treatments per day), duration (e.g., number of minutes that the PEMF therapy is delivered), or pulsed energy signal strength. In another other example, if the temperature difference decreases, then the PEMF treatment may be decreased.

If the PEMF apparatus 100 determines that no changes to the PEMF treatment is needed, then the method returns to 206. On the other hand, if the PEMF apparatus 100 determines that a change to the PEMF treatment is needed, then the PEMF apparatus 100 may modify the PEMF treatment 214. For example, as described above, if the temperature difference between contralateral body locations increases, then the PEMF treatment may be changed to increase a frequency, duration, and/or pulsed energy signal strength. In another example, if the temperature difference between contralateral body locations decreases, then the PEMF treatment may be changed to decrease a frequency, duration, and/or pulsed energy signal strength. The method may return to 206.

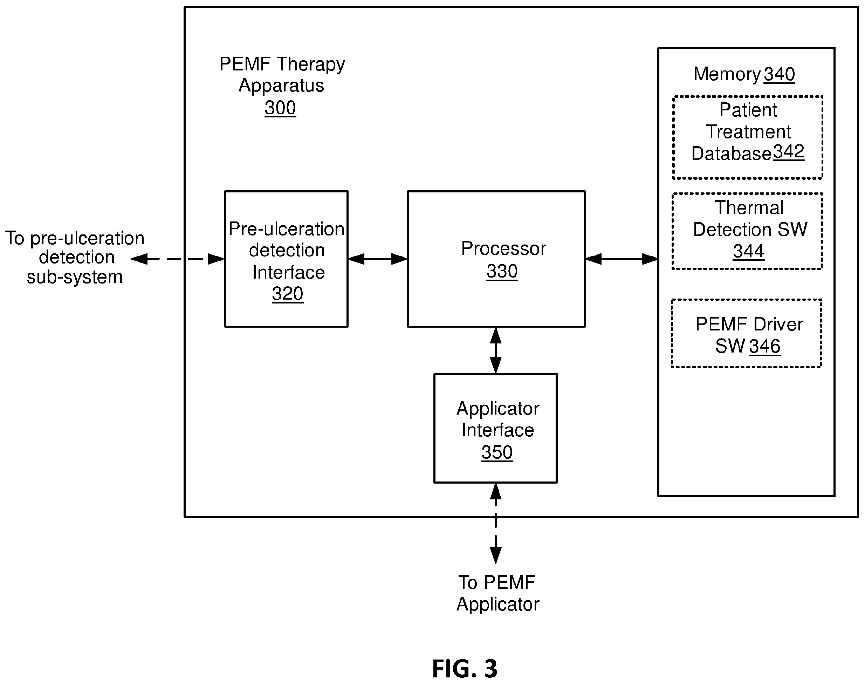

FIG. 3 shows a block diagram of a portion of a PEMF therapy apparatus 300 including an integrated a PEMF delivery sub-system as shown in FIG. 1A. The PEMF therapy device 300 may include a pre-ulceration detection interface 320, a processor 330, a memory 340, and an applicator interface 350.

The pre-ulceration detection interface 320, which is coupled to the processor 330, may be used to interface with any feasible scanning and/or sensing sub-system (or separately provided sensing device as mentioned above). For example, a detection interface 320 may be coupled to and interface with a foot temperature monitoring mat (as a thermal detection interface). In another example, the detection interface 320 may be coupled to and interface with a conforming temperature sensing mat. In still another example, a detection interface 320 may be coupled to and interface with a thermal camera. In yet other examples, the detection interface 320 may be coupled to and interface with any feasible thermal sensing device.

The applicator interface 350, which is also coupled to the processor 330, may be used to interface and control any feasible PEMF applicator, such as PEMF applicator 120. The applicator interface 350 may provide a high-power pulsed electromagnetic field signal to a PEMF applicator. The PEMF applicator, in return, may emit an electromagnetic field, such as a magnetic field, that may treat and penetrate body tissues. In some examples, the applicator interface 350 may include driver circuitry (not shown) to generate the high-power pulsed electromagnetic field signals for the PEMF applicators.

The processor 330, which is also coupled to the detection interface 320, the applicator interface 350, and the memory 340, may be any one or more suitable processors capable of executing scripts or instructions of one or more software programs stored in the PEMF therapy apparatus 300 (such as within memory 340).

The memory 340 may include a patient treatment database 342 that may be used to locally store PEMF treatment protocols for patients. For example, the patient treatment database 342 may include treatment time duration information, number of treatments per day information, pulsed energy signal strength information, or any other feasible treatment information.

The memory 340 may also include a non-transitory computer-readable storage medium (e.g., one or more nonvolatile memory elements, such as EPROM, EEPROM, Flash memory, a hard drive, etc.) that may store the following software modules: a detection software (SW) module 344 to process data from the detection interface 320; and a PEMF Driver SW module 346 to control the high-power pulsed electromagnetic field signal provided by the applicator interface 350.

Each software module may include program instructions that, when executed by the processor 330, may cause the PEMF therapy device 300 to perform the corresponding function(s). Thus, the non-transitory computer-readable storage medium of memory 340 may include instructions for performing all or a portion of the operations described herein.

In general, the processor may analyze data from the pre-ulceration sub-system(s) and/or a separate sensor processor may be used. In some examples the processor 330 may execute the detection SW module 344 to determine the temperature of one or more body locations of a patient. For example, execution of the thermal detection SW module 344 may identify two or more contralaterally-matched body locations of the patient by receiving thermal data (temperature data) from thermal sensors (not shown) coupled to the thermal detection interface 320. In some examples, execution of the thermal detection SW module 344 may determine whether a temperature difference between two or more contralaterally-matched body locations is greater than a threshold. If the temperature is greater than a threshold, then the body location may be associated with a pre-ulcerative lesion. In some other examples, execution of the thermal detection SW module 344 may determine whether the temperature between contralaterally-matched body locations continues to exceed a threshold or whether the temperature between contralaterally-matched body locations no longer exceeds a threshold. In some examples, execution of the thermal detection SW module 344 may cause a thermal scanner to perform multiple thermal scans such as a first (e.g., baseline) thermal scan and subsequent thermal scans.

The processor 330 may execute the PEMF driver SW module 346 to control the energy signals delivered via the applicator interface 350 to one or more PEMF applicators (not shown). For example, execution of the PEMF driver SW module 346 may cause the applicator interface 350 to provide a high-power pulsed electromagnetic field signal based on a thermal information from the thermal detection interface 320. Execution of the PEMF driver SW module 346 may cause the applicator interface 350 to increase or decrease the PEMF therapy delivered through the applicator interface 350. In some cases, execution of the PEMF driver SW module 346 may cause patient therapy information to be stored in the patient treatment database 342. For example, if thermal scan data indicates a worsening of pre-ulcerative conditions (e.g., an increase in a temperature difference between contralaterally-matched body location), then the processor 330 may store an increased PEMF therapy in the patient treatment database 342.

### Examples

As mentioned, diabetic foot ulcers (DFU) have a high annual incidence of about 5% among U.S. Veterans with diabetes, and an annual incidence between 2-5% in the general population. An additional compounding complication leading to DFU is diabetic neuropathy, which initially involves the feet. Symptoms of diabetic neuropathy include increased or decreased sensation resulting from damage of the myelinated and unmyelinated cutaneous nerve fibers. Once clinically presented, treatment of DFUs may involve debridement, offloading, and infection control. However, prevention of DFUs (routine foot screening, proper footwear, and glycemic control) should be considered the primary modality of management. The methods described herein may allow DFU detection prior to ulceration and treatment using PEMF. As described above, detection may include skin temperature monitoring to detect areas of elevated temperature between contralateral areas and spectral imaging, for non-invasively determining levels of tissue oxygenation and changes to microcirculation. These techniques may identify areas of the foot that are at risk of ulceration due to underlying inflammation (e.g., "hot spots", "pre-ulcerative lesions") or poor circulation, with 93-97% sensitivity. The ability to identify localized areas of inflammation gives physicians a warning that, without intervention, the patient will ulcerate. Also described herein for the first time are methods and apparatuses to specifically and actively treat the pre-ulcerous regions.

The apparatuses described herein may be used to self-administer non-thermal, non-ionizing pulsed electromagnetic energy to the target tissue, using, e.g., 27.12 MHz pulses lasting 42 microseconds and delivered at about 1000 times per second. In some examples the system generates an electromagnetic field that is continuously monitored and regulated to ensure consistent dosing. These methods and apparatuses may provide dual-field electromagnetic energy (i.e., high electric and magnetic fields). The therapeutic electromagnetic field may be delivered by means of an applicator (e.g., a pad that is placed against the treatment site).

The effect of PEMF treatment on incidence of diabetic foot ulcers in diabetic patients treating feet was examined. PEMF was prescribed to patients with peripheral neuropathy by their treating physician, throughout their course of treatment contact was made by patient care coordinators as part of the treatment and responses were recorded in an Electronic Reporting Portal (ERP). A retrospective analysis using data collected was performed; the data included patients diagnosed with diabetes who treated their feet and the rate of DFU occurring after beginning treatment (N=196). The same ERP database was queried in three repositories for any incidence of ulceration and manually assessed to determine if the ulcer occurred in the same area as the treatment. Details of the data inquiries are described below. A database of data collected from the Patient Support Program (PPSP) includes voluntary patient self-reported data regarding compliance, pain scores, global impression of change, daily activities, range of motion, perceived inflammation, medication usage, and sleep quality. The PPSP data are recorded in the ERP system. Data collected from the ERP between July 2017 and March 2019 was filtered to identify a sub-set of patients with reported neuropathy of various etiologies in one or both feet. This sub-set of patients were contacted by Patient Care Coordinators to verify if they were also diagnosed with diabetes mellitus (type I or type II); those patients diagnosed with diabetes were used in this retrospective analysis. In total, data was a collected from a total of 196 patients reported treating at least 30 days were examined.

A second database query was performed to determine the general rate of ulceration among the overall treating population, with an agnostic diabetes diagnosis. The ERP system records both medical inquiries and medical complaints, where trained patient care coordinators, nurses, or nurse practitioners can enter patient supplied data. A search for "ulcer" was performed to query the ERP database in three separate repositories, quality of life inquiry (QLI) (N =7,945), medical inquiries and medical complaints (N > 18,000). Data were manually inspected to determine if ulceration occurred in the same area of patient treatment.

The first set of retrospective data included diabetic patients treating feet for neuropathy (N=196 patients). Within this dataset, there were no identified incidences of diabetic foot ulcers for the duration of treatment with the apparatus. Given the included population were diabetic, it would be expected that between 2-6% (or 4-10 patients) within this patient population would have experienced a DFU, given the incidence within the overall diabetic patient population nationwide (CITE). Surprisingly, the retrospective data show no DFU. The expected incidence of DFU among diabetic Veterans is around 5% per year, it would be expected that approximately 10 patients within the dataset would have experienced a DFU while undergoing treatment. As no diabetic patients ulcerated during treatment, suggesting that PEMF treatment is preventing ulceration from occurring.

Data collected from the ERP database using the "ulcer" keyword search resulted in 80 results with "ulcer" as part of the QLI, medical inquiry, or medical complaint repositories. Of these, only one entry included ulceration in the treatment area.

While the original search encompassed over 18,000 patients, 449 patients were treating feet for neuropathy. A single incidence of a patient developing an ulcer in the treating patient population is extremely surprising, given the 2% incidence among diabetics each year. The recurrence of a DFU among patients with a previous ulcer is close to 40% in the first year. As such, more patients would be expected to report recurrent ulcerations when being treated for diabetic complications, including neuropathy and DFU. Considering the low incidence of ulcers in active PEMF treatment areas reported in the data, PEMF appears to prevent ulceration from forming or recurring in this high-risk population. Detecting areas or pre-ulcerative lesions and treating by centering that region on the highest area of therapy would likely decrease the likelihood of the emergence or recurrence of a DFU. Furthermore, it would be beneficial to provide methods and apparatuses for targeting pre-ulcerous lesions for the surprisingly effective PEMF therapy, as this would reduce the overall need for PEMF and/or the treatment times, and amount of energy applied.

### RNA Sequencing identifies PEMF Wound Treatment-Responsive Pathways

The effect of PEMF treatment on wound healing related genetic pathways was examined utilizing Next Generation Sequencing (NGS) of an in vivo study involving an animal model. Two pigs *(Sus scrofa)* were used in this pilot study. For each animal, three quadrants of twelve 2x2 cm full-thickness wounds were made on each animal. Throughout the course of treatment, 4 mm punch biopsies were taken at wounding and days 1, 3, 5, 7, 10, and 21 and snap frozen for subsequent RNA analysis. Animals were monitored for possible infections at wound sites.

Each test subject was randomly assigned to receive active or sham PEMF Therapy treatment to two quadrants located on the animal flanks where the initial tissue biopsies were performed by a researcher blind to the type of device (active or sham). A third quadrant was left untreated. Test subjects received twice daily treatments for 30 minutes. RNA isolation was performed on 5 mm punch biopsies using the RNeasy Mini Kit (QIAGEN Cat #74106) and QIAshredder homogenization columns (QIAGEN Cat # 79654) following manufacturer instructions. Following extraction, samples were quantified using RNA screentape (Agilent, Cat #5067-5578) on the Agilent Tapestation 1500 to determine quality, quantity, and to detect any DNA contamination. RNAseq libraries for sequencing were prepared using the Kapa RNA HyperPrep kit with RiboErase (Roche, Cat# KK8560) using unique dual indexes (Roche, Cat# KK8727) suitable for multiplexing on an Illumina NovaSeq. Libraries were sequenced on an Illumina NovaSeq 6000 using a 2 x 150 bp S4 flowcell (Illumina, cat # 20044417) targeting a depth of ~15M total reads per library.

The *Sus scrofa* genome was assembled through a custom pipeline using STAR v 2.7.5a using the GenomeGenerate function. Genome files were downloaded from UCSC Genome Browser (assembly ID: susScr11). Sequencing FASTQ files from the RNAseq libraries were assessed for quality using FASTQC and low-quality reads were removed (Q<30). High-quality FASTQ reads were assessed using Picard v 2.23.3 CollectRnaSeqMetrics and then aligned to the in-house built genome using STAR to generate gene count files. Gene counts were analyzed for differential expression using the South Dakota State integrated web application for differential expression and pathway analysis of RNA-Seq data (iDEP.951).

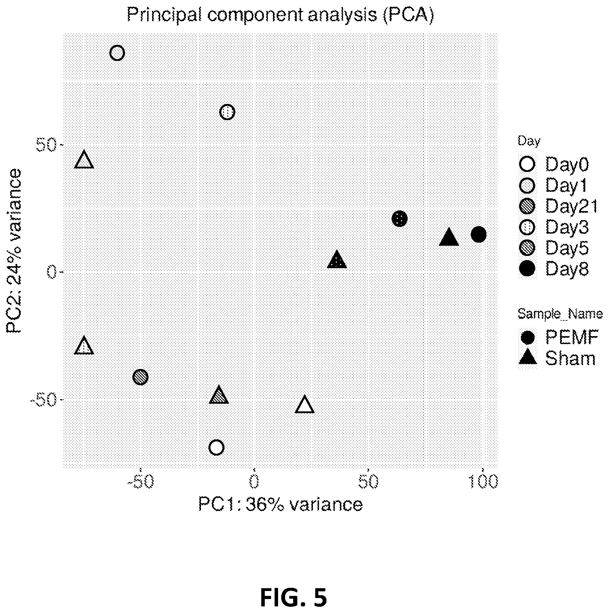

RNAseq utilizing NGS measures the total number of RNA transcripts present in a sample and is an unbiased method to survey overall expression changes. An average of 10M total reads per sample were analyzed. RNA expression data was clustered using a Principal Component Analysis (FIG. 5). Data aggregated by timepoint, which is expected given the time-dependent pathways involved in wound healing. Differential expression analyses using DESeq2 resulted in 134 down-regulated genes in PEMF compared to sham and 58 up-regulated genes compared to sham with a minimum fold change of 2, and a false discovery cutoff of 0.1.

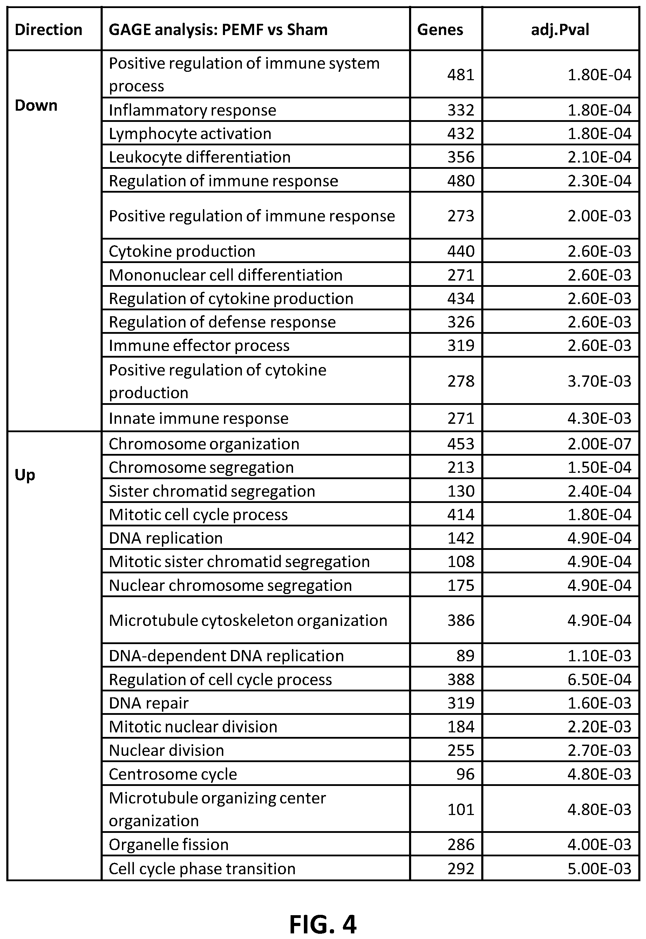

A generally applicable gene-set enrichment (GAGE) for pathway analysis conducted through iDEP.951 resulted in the down-regulation of regulatory pathways including the inflammatory response and innate immune response at a significance level of < 1x10³ (see, FIG. 4, Table 1) in PEMF treated samples, suggesting the PEMF as described herein may be reducing localized inflammation that leads to pre-ulcerative conditions. Additionally, the GAGE data also show up-regulation of pathways involved in cellular replication, indicating a positive impact in proliferation at a significance level of < 5x10³ (FIG. 4) in PEMF treated samples.

The RNA sequencing data indicate that over the course of treatment with PEMF, inflammatory pathways are downregulated. While previous studies in vivo studies have indicated a potential relationship between therapy and inflammation, this is the first in vivo study to show downregulation of multiple inflammatory pathways in an in vitro model. One of the primary indicators of a pre-ulcerative lesion is an increase in inflammation, the ability of PEMF to down-regulate entire pathways involved with this process suggest direct treatment of areas of inflammation would be reduced, thereby preventing ulcer emergence.

It should be appreciated that all combinations of the foregoing concepts and additional concepts discussed in greater detail below (provided such concepts are not mutually inconsistent) are contemplated as being part of the inventive subject matter disclosed herein and may be used to achieve the benefits described herein.

The process parameters and sequence of steps described and/or illustrated herein are given by way of example only and can be varied as desired. For example, while the steps illustrated and/or described herein may be shown or discussed in a particular order, these steps do not necessarily need to be performed in the order illustrated or discussed. The various example methods described and/or illustrated herein may also omit one or more of the steps described or illustrated herein or include additional steps in addition to those disclosed.

Any of the methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to control perform any of the steps, including but not limited to: displaying, communicating with the user, analyzing, modifying parameters (including timing, frequency, intensity, etc.), determining, alerting, or the like. For example, any of the methods described herein may be performed, at least in part, by an apparatus including one or more processors having a memory storing a non-transitory computer-readable storage medium storing a set of instructions for the processes(s) of the method.

While various embodiments have been described and/or illustrated herein in the context of fully functional computing systems, one or more of these example embodiments may be distributed as a program product in a variety of forms, regardless of the particular type of computer-readable media used to actually carry out the distribution. The embodiments disclosed herein may also be implemented using software modules that perform certain tasks. These software modules may include script, batch, or other executable files that may be stored on a computer-readable storage medium or in a computing system. In some embodiments, these software modules may configure a computing system to perform one or more of the example embodiments disclosed herein.

As described herein, the computing devices and systems described and/or illustrated herein broadly represent any type or form of computing device or system capable of executing computer-readable instructions, such as those contained within the modules described herein. In their most basic configuration, these computing device(s) may each comprise at least one memory device and at least one physical processor.

The term "memory" or "memory device," as used herein, generally represents any type or form of volatile or non-volatile storage device or medium capable of storing data and/or computer-readable instructions. In one example, a memory device may store, load, and/or maintain one or more of the modules described herein. Examples of memory devices comprise, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In addition, the term "processor" or "physical processor," as used herein, generally refers to any type or form of hardware-implemented processing unit capable of interpreting and/or executing computer-readable instructions. In one example, a physical processor may access and/or modify one or more modules stored in the above-described memory device. Examples of physical processors comprise, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor.

Although illustrated as separate elements, the method steps described and/or illustrated herein may represent portions of a single application. In addition, in some embodiments one or more of these steps may represent or correspond to one or more software applications or programs that, when executed by a computing device, may cause the computing device to perform one or more tasks, such as the method step.

In addition, one or more of the devices described herein may transform data, physical devices, and/or representations of physical devices from one form to another. Additionally or alternatively, one or more of the modules recited herein may transform a processor, volatile memory, non-volatile memory, and/or any other portion of a physical computing device from one form of computing device to another form of computing device by executing on the computing device, storing data on the computing device, and/or otherwise interacting with the computing device.

The term "computer-readable medium," as used herein, generally refers to any form of device, carrier, or medium capable of storing or carrying computer-readable instructions. Examples of computer-readable media comprise, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

A person of ordinary skill in the art will recognize that any process or method disclosed herein can be modified in many ways. The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed.

The various exemplary methods described and/or illustrated herein may also omit one or more of the steps described or illustrated herein or comprise additional steps in addition to those disclosed. Further, a step of any method as disclosed herein can be combined with any one or more steps of any other method as disclosed herein.

The processor as described herein can be configured to perform one or more steps of any method disclosed herein. Alternatively, or in combination, the processor can be configured to combine one or more steps of one or more methods as disclosed herein.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive, and may be expressed as "consisting of" or alternatively "consisting essentially of" the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The foregoing description is provided primarily for exemplary purposes and should not be interpreted to limit the scope of the invention as it is set forth in the claims.

## Claims

1. A pulsed electromagnetic field, PEMF, system (100) comprising:
a detection sub-system (125, 130) configured to perform a first scan to determine optical properties and/or temperatures of two or more body locations and determine a location of a pre-ulcerative lesion prior to ulcer formation by determining an optical property and/or temperature difference between two or more contralaterally-matched body locations;
a PEMF generator (110) configured to generate a PEMF treatment output; and
a PEMF applicator (120) coupled to the PEMF generator and configured to deliver a PEMF treatment to the determined location of the pre-ulcerative lesion.

2. The PEMF system of claim 1, further comprising a processor (330) configured to monitor the pre-ulcerative lesion over time and to adjust the PEMF treatment based on a progression of the pre-ulcerative lesion.

3. The PEMF system of claim 1, wherein the detection sub-system comprises a tissue oximetry detection sub-system.

4. The PEMF system of claim 1, wherein the detection sub-system comprises a thermal detection sub-system (130).

5. The PEMF system of claim 4, wherein the thermal detection sub-system (130) is configured to determine temperatures of two or more body locations.

6. The PEMF system of claim 4, wherein the thermal detection sub-system (130) is configured to determine a temperature difference between two or more contralaterally-matched body locations.

7. The PEMF system of claim 6, wherein the PEMF system is configured to determine whether the temperature difference is greater than a threshold to determine the location of the pre-ulcerative lesion.

8. The PEMF system of claim 1, wherein the PEMF generator (110) is configured to generate 27.12 MHz pulses lasting between 35-50 microseconds and delivered at between 800-1200 times per second.

9. The PEMF system of claim 1, wherein the detection sub-system (125, 130) comprises one or more of: a foot temperature monitoring mat, a conforming temperature sensing mat, a thermal camera.

10. The PEMF system of claim 1, wherein the detection sub-system (125, 130) includes an optical detector configured to sense absorption and/or reflectance of light in one or more specific wavelengths to assess the risk of ulcer development.

11. The PEMF system of claim 1, wherein the detection sub-system (125, 130) is further configured to perform a subsequent scan of the location of the pre-ulcerative lesion after a predetermined time after the PEMF treatment, and wherein the processor is configured to compare the first scan to the subsequent scan.

12. The PEMF system of claim 11, wherein the processor (330) is configured to increase a duration of the PEMF treatment based on the comparison of the first scan to the subsequent scan.

## Patentansprüche

1. Gepulstes elektromagnetisches Feld-, PEMF-, System (100), umfassend:
ein Detektionssubsystem (125, 130), das dazu konfiguriert ist, einen ersten Scan durchzuführen, um optische Eigenschaften und/oder Temperaturen von zwei oder mehr Körperstellen zu ermitteln und eine Stelle einer präulzerativen Läsion vor der Ulkusbildung durch Ermitteln einer optischen Eigenschaft und/oder Temperaturdifferenz zwischen zwei oder mehr kontralateral übereinstimmenden Körperstellen zu ermitteln;
einen PEMF-Generator (110), der dazu konfiguriert ist, eine PEMF-Behandlungsausgabe zu erzeugen; und
einen PEMF-Applikator (120), der an den PEMF-Generator gekoppelt und dazu konfiguriert ist, eine PEMF-Behandlung an die ermittelte Stelle der präulzerativen Läsion abzugeben.

2. PEMF-System nach Anspruch 1, ferner umfassend einen Prozessor (330), der dazu konfiguriert ist, die präulzerative Läsion im Verlauf der Zeit zu überwachen und die PEMF-Behandlung, basierend auf einem Fortschreiten der präulzerativen Läsion, anzupassen.

3. PEMF-System nach Anspruch 1, wobei das Detektionssubsystem ein Gewebeoximetrie-Detektionssubsystem umfasst.

4. PEMF-System nach Anspruch 1, wobei das Detektionssubsystem ein thermisches Detektionssubsystem (130) umfasst.

5. PEMF-System nach Anspruch 4, wobei das thermische Detektionssubsystem (130) dazu konfiguriert ist, Temperaturen von zwei oder mehr Körperstellen zu ermitteln.

6. PEMF-System nach Anspruch 4, wobei das thermische Detektionssubsystem (130) dazu konfiguriert ist, eine Temperaturdifferenz zwischen zwei oder mehr kontralateral übereinstimmenden Körperstellen zu ermitteln.

7. PEMF-System nach Anspruch 6, wobei das PEMF-System dazu konfiguriert ist, zu ermitteln, ob die Temperaturdifferenz größer als eine Schwelle ist, um die Stelle der präulzerativen Läsion zu ermitteln.

8. PEMF-System nach Anspruch 1, wobei der PEMF-Generator (110) dazu konfiguriert ist, 27,12-MHz-Pulse zu erzeugen, die zwischen 35-50 Mikrosekunden dauern und zwischen 800-1200 Mal pro Sekunde abgegeben werden.

9. PEMF-System nach Anspruch 1, wobei das Detektionssubsystem (125, 130) eines oder mehrere von Folgendem umfasst: eine Fußtemperaturüberwachungsmatte, eine konforme Temperaturerfassungsmatte, eine Wärmekamera.

10. PEMF-System nach Anspruch 1, wobei das Detektionssubsystem (125, 130) einen optischen Detektor beinhaltet, der dazu konfiguriert ist, Absorption und/oder Reflexion von Licht in einer oder mehreren spezifischen Wellenlängen zu erfassen, um das Risiko einer Ulkusentwicklung zu bewerten.

11. PEMF-System nach Anspruch 1, wobei das Detektionssubsystem (125, 130) ferner dazu konfiguriert ist, einen nachfolgenden Scan der Stelle der präulzerativen Läsion nach einer vorbestimmten Zeit nach der PEMF-Behandlung durchzuführen, und wobei der Prozessor dazu konfiguriert ist, den ersten Scan mit dem nachfolgenden Scan zu vergleichen.

12. PEMF-System nach Anspruch 11, wobei der Prozessor (330) dazu konfiguriert ist, eine Dauer der PEMF-Behandlung basierend auf dem Vergleich des ersten Scans mit dem nachfolgenden Scan zu verlängern.

## Revendications

1. Système (100) à champ électromagnétique pulsé, CEMP, comprenant :
un sous-système de détection (125, 130) conçu pour effectuer un premier balayage afin de déterminer les propriétés optiques et/ou les températures de deux emplacements corporels ou plus et de déterminer l'emplacement d'une lésion pré-ulcéreuse avant la formation d'un ulcère en déterminant une propriété optique et/ou une différence de température entre deux emplacements corporels appariés ou plus de façon controlatérale ;
un générateur de CEMP (110) conçu pour générer une sortie de traitement par CEMP ; et
un dispositif d'application de CEMP (120) couplé au générateur de CEMP et conçu pour administrer un traitement par CEMP à l'emplacement déterminé de la lésion pré-ulcéreuse.

2. Système à CEMP selon la revendication 1, comprenant en outre un processeur (330) configuré pour surveiller la lésion pré-ulcéreuse au fil du temps et pour ajuster le traitement par CEMP sur la base de la progression de la lésion pré-ulcéreuse.

3. Système à CEMP selon la revendication 1, ledit sous-système de détection comprenant un sous-système de détection d'oxymétrie de tissu.

4. Système à CEMP selon la revendication 1, ledit sous-système de détection comprenant un sous-système de détection thermique (130).

5. Système à CEMP selon la revendication 4, ledit sous-système de détection thermique (130) étant configuré pour déterminer les températures de deux emplacements corporels ou plus.

6. Système à CEMP selon la revendication 4, ledit sous-système de détection thermique (130) étant configuré pour déterminer la différence de température entre deux emplacements corporels appariés de façon controlatérale ou plus.

7. Système à CEMP selon la revendication 6, ledit système à CEMP étant configuré pour déterminer si la différence de température est supérieure à un seuil de manière à déterminer l'emplacement de la lésion pré-ulcéreuse.

8. Système à CEMP selon la revendication 1, ledit générateur de CEMP (110) étant configuré pour générer des impulsions de 27,12 MHz d'une durée comprise entre 35 et 50 microsecondes et délivrées entre 800 à 1200 fois par seconde.

9. Système à CEMP selon la revendication 1, ledit sous-système de détection (125, 130) comprenant un ou plusieurs éléments parmi : un tapis de surveillance de la température des pieds, un tapis de détection de température conforme, une caméra thermique.

10. Système à CEMP selon la revendication 1, ledit sous-système de détection (125, 130) comprenant un détecteur optique configuré pour détecter l'absorption et/ou la réflectance de la lumière dans une ou plusieurs longueurs d'onde spécifiques de manière à évaluer le risque de développement d'un ulcère.

11. Système à CEMP selon la revendication 1, ledit sous-système de détection (125, 130) étant en outre configuré pour effectuer un balayage ultérieur de l'emplacement de la lésion pré-ulcéreuse après un temps prédéfini après le traitement par CEMP, ledit processeur étant configuré pour comparer le premier balayage au balayage ultérieur.

12. Système à CEMP selon la revendication 11, ledit processeur (330) étant configuré pour augmenter la durée du traitement par CEMP sur la base de la comparaison du premier balayage au balayage ultérieur.
